# FASCICULE DE BREVET EUROPEEN

(11) **EP 2 090 759 B1**
(45) Date de publication et mention de la délivrance du brevet: **22.12.2010**
(21) Numéro de dépôt: 09152486.8
(22) Date de dépôt: 10.02.2009
(51) Int. Cl.: F01M 11/04, F01M 11/10, B03C 1/28, F16N 29/04, G01N 15/04, G01N 15/06, G01V 3/02

(54) **Bouchon magnétique à pré-signalisation**
Magnetzapfen mit Vorwarnfunktion
Pre-signalling magnetic plug

(30) Priorité: 13.02.2008 FR 0850894
(43) Date de publication de la demande: 19.08.2009
(73) Titulaire: Turbomeca, 64510 Bordes (FR)
(72) Inventeur: Augros, Philippe, 64160 Serres-Morlaas (FR); Senger, Gérald, 64110 Morlaas (FR)
(74) Mandataire: Balesta, Pierre

(56) Documents cités:
- DE-A1- 19 731 960
- DE-A1-102004 003 559
- JP-A- 2002 286 697
- US-A- 5 402 113
- US-A1- 2005 212 533

## Description

La présente invention concerne le domaine de la détection de l'usure de pièces, telles par exemple mais non nécessairement les pièces rotatives disposées dans un carter d'équipement (ou d'accessoire) ou de moteur d'un aéronef.

La présente invention concerne plus précisément le domaine des bouchons magnétiques, notamment les bouchons magnétiques à signalisation.

De manière plus précise, l'invention porte sur un bouchon magnétique à signalisation destiné à être placé dans un circuit de liquide comportant une première électrode magnétique et une deuxième électrode magnétique isolées l'une de l'autre et destinées à être en contact avec le liquide circulant dans le circuit de liquide lorsque le bouchon magnétique est monté, afin de détecter la présence de particules métalliques susceptibles d'être convoyées par le liquide.

Traditionnellement, un tel bouchon magnétique à signalisation est monté sur un carter contenant des pièces rotatives, tels des engrenages ou des roulements, qui baignent dans ledit liquide, voire pour example DE 197 31 960

De manière connue, la fonction du circuit de liquide est généralement de permettre la lubrification et/ou le refroidissement des pièces rotatives.

Il se trouve que les pièces rotatives sont amenées à s'user au cours de leur vie, cette usure pouvant être normale, par exemple en raison du frottement résultant du contact entre deux roues dentées, ou bien être tout à fait anormale, par exemple en raison de chocs ou frottements intenses entre pièces rotatives dus à des vibrations intenses et anormales se propageant dans le carter, lesquelles peuvent notamment résulter de la casse d'une pièce rotative. La dégradation d'un moteur d'aéronef peut également causer une usure anormale des pièces constitutives du moteur.

Quelle que soit sa cause, l'usure des pièces entraine la formation de particules qui se détachent des pièces et sont entrainées par le liquide dans le circuit de liquide.

Dans la mesure où les pièces rotatives sont généralement métalliques, les particules résultant de l'usure des pièces sont conductrices et se présentent généralement sous la forme de limaille. Qui plus est, les pièces sont le plus souvent réalisées en un métal du type ferromagnétique, comme le fer c'est-à-dire apte à être attiré par un élément magnétique tel un aimant.

De manière connue, le bouchon magnétique à signalisation permet de détecter la présence de telles particules métalliques.

Pour ce faire, les première et deuxième électrodes magnétiques attirent les particules métalliques lors de la circulation du liquide, à la suite de quoi l'accumulation des particules sur l'une et/ou l'autre des électrodes magnétiques tend à former un pont conducteur reliant les électrodes magnétiques.

Dès lors qu'un tel pont conducteur est formé, un circuit électrique associé est à même de détecter la formation de ce pont conducteur entrainant l'envoi d'un signal à un opérateur, par exemple le pilote de l'aéronef.

Lorsqu'un tel bouchon magnétique est disposé pour détecter la présence de particules, la réception d'un signal provenant du bouchon magnétique, également appelée « allumage du bouchon », impose à l'opérateur des restrictions d'utilisation et de réaliser une opération de maintenance afin de déterminer la cause de la présence des particules métalliques. Par exemple dans le cas de la détection de particules dans le circuit de lubrification d'un moteur d'hélicoptère, l'« allumage du bouchon », impose au pilote un atterrissage en sécurité au plus tôt dans le cas d'un monomoteur ou pour les applications multi-moteurs un passage au ralenti du moteur concerné. La mission est affectée et l'hélicoptère devra être immobilisé pendant une durée assez longue pour opération de maintenance.

Au demeurant, on constate souvent des allumages du bouchon magnétique, ne résultant pas d'une usure anormale du moteur (allumages qualifiés d'intempestifs).

C'est le cas par exemple pendant les deux cents premières heures de fonctionnement du moteur, période pendant laquelle on constate l'accumulation de particules métalliques sur le bouchon alors que les pièces rotatives ne sont pas anormalement usées. La cause de la présence de ces particules métalliques en début de vie du moteur est généralement due à un effet de rodage ou un nettoyage imparfait des pièces du moteur lorsque ce dernier sort des chaines de production du fabricant ou. Des particules métalliques peuvent donc être présentes dans le circuit d'huile en l'absence d'usure anormale.
C'est aussi le cas après la période de rodage, ou en fonctionnement normal les particules issues de l'usure normale du moteur s'accumulent au bouchon magnétique pour finir par produire son allumage.

Bien que ces allumages de voyant ne soient pas causés par un fonctionnement anormal du moteur, ils sont pris en compte de la même façon avec les mêmes conséquences opérationnelles et de maintenance.

Traditionnellement, une solution pour pallier à ces allumages intempestifs et éviter d'immobiliser l'hélicoptère, on brûle les particules accumulées sur le bouchon.

Un inconvénient est que l'on perd l'information sur la raison de l'allumage du bouchon magnétique. En d'autres termes, ce procédé qui consiste à bruler les particules, ne permet pas, lors d'une opération de maintenance, de valider si la présence des particules sur le bouchon était due à une pollution antérieure ou bien à une usure anormale et même d'identifier les pièces par analyse de la matière.

On connaît également des systèmes de comptage de particules permettant d'étudier l'évolution en taille et en nombre des particules mais ceux-ci présentent de nombreux inconvénients, à savoir notamment leur prix et leur masse. Ils présentent aussi l'inconvénient de contenir des circuits électroniques dont il convient de s'assurer du fonctionnement correct en conditions opérationnelles.

Un but de la présente invention est de proposer un bouchon magnétique à signalisation simple et peu couteux, permettant d'une part de détecter de manière précoce la présence de particules métalliques sans restreindre immédiatement les conditions d'utilisation, et d'autre part de maintenir toutes les capacités d'investigations en maintenance.

L'invention atteint son but par le fait que le bouchon selon l'invention comporte en outre une électrode intermédiaire disposée entre les première et deuxième électrodes magnétiques tout en étant également destinée à être en contact avec le liquide circulant dans le circuit de liquide lorsque le bouchon magnétique est monté, grâce à quoi ledit bouchon permet une détection précoce de la présence de telles particules métalliques.

De préférence, l'électrode intermédiaire est amagnétique.

Lors de l'accumulation de particules métalliques sur les première et deuxième électrodes magnétiques, un pont conducteur principal tend à se former entre ces deux électrodes.

Avant que le pont entre les électrodes magnétiques ne soit formé, il arrive un moment où l'un des amas de particules, présentant une élongation vers l'autre électrode magnétique, entre en contact avec l'électrode intermédiaire.

Il s'ensuit la formation d'un pont intermédiaire conducteur entre l'une des deux électrodes magnétiques et l'électrode intermédiaire avant la formation du pont principal entre les deux électrodes magnétiques.

L'électrode intermédiaire, préférentiellement choisie amagnétique, n'attire pas les particules métalliques et donc ne perturbe pas la formation du pont principal entre les première et deuxième électrodes magnétiques.

La formation du pont intermédiaire conducteur permet de générer un signal intermédiaire distinct et avantageusement préalable à celui généré par la formation du pont principal, grâce à quoi on réalise la détection précoce de la présence de particules métalliques dans le liquide.

La génération d'un signal intermédiaire, également appelé présignalisation, permet de détecter de façon précoce la présence de particules métalliques et de planifier les opérations de maintenance et de surveillance associées.

L'analyse des particules associées à cette détection intermédiaire permet de déterminer l'origine de l'accumulation des particules au plus tôt et entraînera soit le nettoyage du bouchon, soit la prévention d'une dégradation d'un élément du moteur.

Dans le cas d'un aéronef, tel un hélicoptère, la présignalisation précitée est préférentiellement activée uniquement lorsque l'aéronef est au sol afin d'éviter une limitation opérationnelle immédiate. A l'issue de la pré signalisation les particules ainsi accumulées seront analysées a posteriori afin de déterminer s'il s'agit d'un allumage du type intempestif ou pas et dans ce dernier cas de prévenir une dégradation du moteur. La dite invention permet donc d'éviter les limitations opérationnelles dues à des allumages intempestifs sans modifier les performances de détection des électrodes magnétiques.

Avantageusement, les première et deuxième électrodes magnétiques sont séparées l'une de l'autre par une portion isolante, et l'électrode intermédiaire est disposée au moins en partie dans la portion isolante de manière à ne pas être en contact électrique avec les première et deuxième électrodes magnétiques.

L'électrode intermédiaire est donc préférentiellement séparée des première et deuxième électrodes magnétiques par deux couches d'isolant.

Selon un premier mode de réalisation avantageux de l'invention, l'électrode intermédiaire est disposée sur le pourtour de la portion isolante.

De préférence, la portion isolante présente la forme générale d'un cylindre axialement enserré par les première et deuxième électrodes magnétiques, tandis que l'électrode intermédiaire forme un anneau disposé dans la portion isolante à distance des première et deuxième électrodes magnétiques.

On pourra par exemple disposer l'électrode intermédiaire à une distance prédéterminée plus proche de la première électrode magnétique que de la deuxième électrode magnétique afin de calibrer un seuil de présignalisation.

Selon un second mode de réalisation avantageux de l'invention, les première et deuxième électrodes magnétiques ainsi que l'électrode intermédiaire présentent la forme de barreaux ou tiges s'étendant depuis la portion isolante.

Ainsi, les extrémités distales des électrodes sont destinées à baigner dans le liquide circulant dans le circuit d'huile.

Conformément à l'invention, le matériau constituant la portion isolante est de préférence amagnétique.

Avantageusement, les première et deuxième électrodes magnétiques, ainsi que l'électrode intermédiaire comprennent des bornes électriques isolées les unes des autres et destinées à être connectées à un circuit électrique.

Dans le cas du premier mode de réalisation, les bornes s'étendent avantageusement selon la direction longitudinale du bouchon magnétique et peuvent être coaxiales.

Ce circuit électrique est arrangé de manière à générer un signal de détection principal ainsi qu'un présignal conformément à la présente invention.

De manière préférentielle, le circuit de liquide est un circuit d'huile.

L'invention concerne en outre un équipement pour aéronef muni d'au moins un bouchon magnétique selon l'invention.

L'invention concerne enfin un moteur pour aéronef comportant un circuit d'huile muni d'un bouchon magnétique selon l'invention.

L'invention sera mieux comprise et ses avantages apparaîtront mieux à la lecture de la description qui suit, de modes de réalisation indiqués à titre d'exemples non limitatifs. La description se réfère aux dessins annexés sur lesquels :
- la figure **1** représente de manière schématique un élément d'un circuit liquide sur lequel est monté un bouchon magnétique selon le premier mode de réalisation de l'invention, un pont intermédiaire de particules étant formé entre la première électrode magnétique et l'électrode intermédiaire ;
- la figure **2** représente l'élément de la figure **1** pour lequel un pont principal de particules est formé entre les première et deuxième électrodes magnétiques ; et
- la figure **3** représente de manière schématique un élément d'un circuit liquide sur lequel est monté un bouchon magnétique selon le second mode de réalisation de l'invention, un pont intermédiaire étant formé entre la première électrode magnétique et l'électrode intermédiaire.

Les bouchons magnétiques qui vont être décrits ci-après sont par exemple, mais non exclusivement, destinés à être montés sur un carter d'un turbomoteur d'hélicoptère **9.** Ce bouchon magnétique peut être aussi monté sur tout autre équipement **8** ou carter comportant un circuit de liquide, tel par exemple un circuit d'huile.

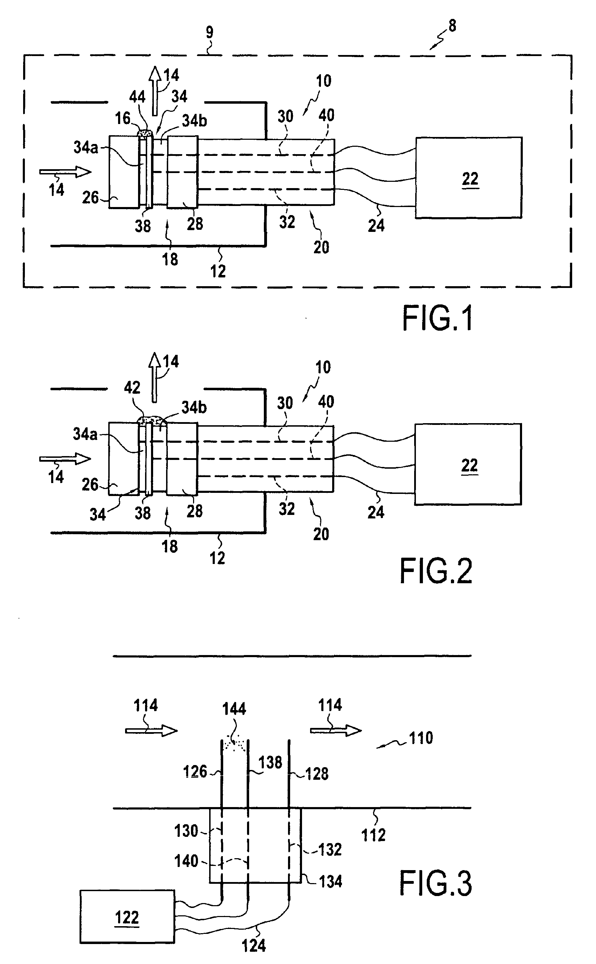

A l'aide des figures **1** et **2** on va tout d'abord décrire un premier mode de réalisation du bouchon magnétique à signalisation **10** selon l'invention.

Le bouchon magnétique **10** est monté sur un carter **12** d'un turbomoteur (non représenté ici). Ce carter **12** enferme des pièces rotatives constitutives du turbomoteur : notamment des engrenages, roulements et arbres permettant d'entrainer les rotors en rotation.

Les éléments rotatifs baignent dans un circuit de liquide, en l'espèce un circuit d'huile dont le sens d'écoulement est schématisé par les flèches **14.**

Comme on l'a déjà expliqué ci-dessus, le circuit d'huile est susceptible de véhiculer des particules métalliques **16** du type ferromagnétique, notamment générées par l'usure des pièces rotatives.

Le bouchon métallique **10** selon le premier mode de l'invention comporte une première portion **18** s'étendant vers l'intérieur du carter **12** tout en baignant dans le circuit de liquide **14,** ainsi qu'une seconde portion **20,** opposée à la première, s'étendant vers l'extérieur du carter **12.**

Bien évidemment, le bouchon magnétique **10** réalise une étanchéité entre l'intérieur et l'extérieur du carter de sorte que l'huile ne fuit pas à l'endroit du bouchon magnétique **10.** En pratique, le bouchon magnétique **10** peut comprendre un filetage de manière à être vissé au carter **12.**

Comme on le voit sur les figures **1** et **2****,** le bouchon magnétique **10,** lorsqu'il est monté, est connecté à un circuit électrique **22** par l'intermédiaire de câbles électriques **24** émergeant de la seconde portion **20** du bouchon magnétique **10.**

La fonction du circuit électrique **22** est de signaler la présence d'un pont de particules conductrices **16** dans le circuit d'huile **14.** Pour ce faire, on pourra utiliser tout type de composant de signalisation adéquat.

Le bouchon magnétique **10** comporte une première électrode magnétique **26,** en l'espèce disposée à l'extrémité du bouchon magnétique **10** qui est opposée à la seconde portion **20,** ainsi qu'une deuxième électrode magnétique **28** disposée entre la première électrode magnétique **26** et la seconde portion **20.**

Comme on le comprend à l'aide des figures **1** et **2****,** les première et deuxième électrodes magnétiques **26,28** sont en contact avec l'huile circulant dans le circuit d'huile lorsque le bouchon magnétique est monté.

Dans l'exemple représenté ici, considérées selon le sens d'écoulement de l'huile, la première électrode magnétique **26** est disposée en amont de la deuxième électrode magnétique **28.**

Par « électrode magnétique » on entend un conducteur électrique qui est aussi apte à attirer les particules métalliques.

Chacune des première et deuxième électrodes magnétiques **26, 28** comprend une borne électrique isolée de l'autre, ces bornes étant respectivement référencées **30,32,** et logées à l'intérieur du bouchon magnétique **10** tout en s'étendant selon sa direction longitudinale depuis la première portion **18** vers la seconde portion **20** de manière à pouvoir être reliées aux câbles **24.**

Par ailleurs, les première et deuxième électrodes magnétiques **26,28** sont électriquement isolées l'une de l'autre.

De préférence, les première et deuxième électrodes **26** et **28** sont séparées l'une de l'autre, de préférence selon la direction longitudinale du bouchon magnétique **10,** par une portion isolante **34,** qui est de préférence amagnétique.

Conformément à la présente invention, le bouchon magnétique à signalisation **10** comporte en outre une électrode intermédiaire **38** disposée entre les première et deuxième électrodes magnétiques **26,28** tout en étant également destinée à être en contact avec l'huile circulant dans le circuit d'huile **14.**

Plus précisément, l'électrode intermédiaire **38** est préférentiellement disposée en partie dans la portion isolante **34,** de préférence sur son pourtour, de manière à ne pas être en contact électrique avec les première et deuxième électrodes magnétiques **26,28.**

L'électrode intermédiaire **38** est donc séparée de la première électrode magnétique **26** par une première couche d'isolant **34**a, et de la deuxième électrode magnétique **28** par une seconde couche d'isolant **34**b.

En l'espèce, l'électrode intermédiaire **38** forme un anneau dont la surface périphérique extérieure est en contact avec l'huile lorsque le bouchon **10** est monté.

De manière similaire aux électrodes magnétiques **26,28,** l'électrode intermédiaire **36** préférentiellement amagnétique comprend une borne électrique **40,** similaire aux bornes **30,32** des électrodes magnétiques, isolée des autres bornes et destinée à être connectée au circuit électrique **22** par l'intermédiaire d'un câble **24.**

En fonctionnement, les particules métalliques **16** susceptibles d'être convoyées par le flux d'huile se fixent, par magnétisme, de préférence sur la première électrode magnétique du fait de sa disposition en amont par rapport au flux d'huile.

Un pont principal **42** tend donc à se former entre les première et deuxième électrodes magnétiques **26,28.** Lorsqu'il est formé, le pont principal relie électriquement les première et deuxième électrodes magnétiques **26,28** comme on l'a représenté sur la figure **2****,** à la suite de quoi le circuit électrique **22** génère un signal indiquant la présence de particules métalliques dans le circuit d'huile.

Avant la formation du pont principal **42,** il arrive un moment, représenté sur la figure 1, où l'amas de particules métalliques s'étendant depuis la première électrode magnétique touche l'électrode intermédiaire **38,** par quoi un pont intermédiaire **44** conducteur se forme entre la première électrode magnétique **26** et l'électrode intermédiaire **38.** Il s'ensuit la génération d'un pré-signal par le circuit électrique **22.**

Comme on le voit sur les figures, l'électrode intermédiaire **38** présente de préférence une largeur axiale sensiblement plus petite que celles des première et deuxième électrodes magnétiques, de manière que la formation du pont intermédiaire **44** ne vienne pas perturber la formation du pont principal **42** entre les première et deuxième électrodes magnétiques.

Au surplus, le fait que l'électrode intermédiaire **38** soit préférentiellement non magnétique, permet également de ne pas trop perturber la formation du pont principal **42.**

A l'aide de la figure **3** on va maintenant décrire un second mode de réalisation d'un bouchon magnétique **110** selon l'invention.

Les organes similaires à ceux du premier mode de réalisation portent la même référence numérique augmentée de la valeur cent.

Le bouchon magnétique **110** de la figure **3** comporte également une première électrode magnétique **126,** une deuxième électrode magnétique **128** séparées l'une de l'autre, tout en étant destinées à être en contact avec l'huile circulant dans le circuit d'huile **114.**

Le bouchon magnétique **110** comporte en outre, conformément à l'invention, une électrode intermédiaire **138** disposée entre les première et deuxième électrodes magnétiques **126,128** tout en étant également destinée à être en contact avec l'huile lorsque le bouchon magnétique **110** est monté, par exemple à un carter **112.**

Dans le mode de réalisation représenté sur la figure **3****,** les première et deuxième électrodes magnétiques **126, 128,** ainsi que l'électrode intermédiaire **138** présentent la forme de barreaux ou tiges s'étendant depuis une portion isolante **134** vers l'intérieur du carter **112.**

Chacune des électrodes magnétiques et intermédiaire est reliée à un circuit électrique **122** par l'intermédiaire de câbles **124,** ces derniers étant reliés à des bornes **130, 132** et **140,** similaires à celles décrites dans le premier mode de réalisation.

Comme on le voit sur la figure **3****,** considérées selon le sens d'écoulement de l'huile, la première électrode magnétique **126** est disposée en amont de la deuxième électrode magnétique **128,** cette dernière étant quant à elle disposée en aval de l'électrode intermédiaire **138.**

Sur la figure **3** on a également représenté un pont intermédiaire **144** de particules métalliques formé entre la première électrode magnétique **126** et l'électrode intermédiaire **138**. II s'ensuit la génération d'un pré-signal par le circuit électrique **122.**

De manière similaire au premier mode de réalisation, un pont principal peut être formé entre la première électrode magnétique **126** et la deuxième électrode magnétique **128,** conduisant à la génération d'un signal par le circuit électrique **122.**

## Revendications

1. Bouchon magnétique à signalisation (10, 110) destiné à être placé dans un circuit de liquide (14, 114) comportant une première électrode magnétique (26, 126) et une deuxième électrode magnétique (28, 128) isolées l'une de l'autre et destinées à être en contact avec le liquide circulant dans le circuit de liquide lorsque le bouchon magnétique (10, 110) est monté, afin de détecter la présence de particules métalliques du type ferromagnétique (16, 116) susceptibles d'être convoyées par le liquide, ledit bouchon étant **caractérisé en ce qu'**il comporte en outre une électrode intermédiaire préférentiellement amagnétique (38, 138) disposée entre les première et deuxième électrodes magnétiques tout en étant également destinée à être en contact avec le liquide circulant dans le circuit de liquide lorsque le bouchon magnétique est monté, grâce à quoi ledit bouchon (10, 110) permet une détection précoce de la présence de telles particules métalliques.

2. Bouchon magnétique selon la revendication **1, caractérisé en ce que** les première et deuxième électrodes magnétiques (26, 126 ; 28, 128) sont séparées l'une de l'autre par une portion isolante (34, 134), et **en ce que** l'électrode intermédiaire (38, 138) est disposée au moins en partie dans la portion isolante (34, 134) de manière à ne pas être en contact électrique avec les première et deuxième électrodes magnétiques.

3. Bouchon magnétique selon la revendication **2, caractérisé en ce que** l'électrode intermédiaire (38) est disposée sur le pourtour de la portion isolante (34).

4. Bouchon magnétique selon la revendication **2, caractérisé en ce que** les première et deuxième électrodes magnétiques (126, 128) ainsi que l'électrode intermédiaire (138) présentent la forme de barreaux s'étendant depuis la portion isolante (134).

5. Bouchon magnétique selon l'une quelconque des revendications **2** à **4, caractérisé en ce que** le matériau constituant la portion isolante (34, 134) est amagnétique.

6. Bouchon magnétique selon l'une quelconque des revendications **1** à **5, caractérisé en ce que** les première et deuxième électrodes magnétiques, ainsi que l'électrode intermédiaire comprennent des bornes électriques (30, 32, 40, 130, 132, 140) isolées les unes des autres et destinées à être connectées à un circuit électrique (22, 122).

7. Bouchon magnétique selon l'une quelconque des revendications **1** à **6, caractérisé en ce que** le circuit de liquide (14, 114) est un circuit d'huile.

8. Equipement pour aéronef muni d'au moins un bouchon magnétique selon l'une quelconque des revendications **1** à **7.**

9. Moteur (9) pour aéronef comportant un circuit d'huile muni d'un bouchon magnétique selon l'une quelconque des revendications **1** à **7.**

## Claims

1. A signaling magnetic plug (10, 110) for placing in a liquid circuit (14, 114), the plug having a first magnetic electrode (26, 126) and a second magnetic electrode (28, 128) that are insulated from each other and that are designed to be in contact with the liquid flowing in the liquid circuit when the magnetic plug (10, 110) is mounted, for the purpose of detecting the presence of metal particles of ferromagnetic type (16, 116) that might be conveyed by the liquid, said plug being **characterized in that** it further comprises a preferably non-magnetic intermediate electrode (38, 138) located between the first and second magnetic electrodes, while also being designed to be in contact with the liquid flowing in the liquid circuit when the magnetic plug is mounted, whereby said plug (10, 110) enables the presence of such metal particles to be detected early.

2. A magnetic plug according to claim 1, **characterized in that** the first and second magnetic electrodes (26, 126; 28, 128) are separated from each other by an insulating portion (34, 134), and **in that** the intermediate electrode (38, 138) is disposed at least in part in the insulating portion (34, 134) so that it is not in electrical contact with either of the first and second magnetic electrodes.

3. A magnetic plug according to claim 2, **characterized in that** the intermediate electrode (38) is disposed around the periphery of the insulating portion (34).

4. A magnetic plug according to claim 2, **characterized in that** the first and second magnetic electrodes (126, 128) and the intermediate electrode (138) are in the form of bars projecting from the insulating portion (134).

5. A magnetic plug according to any one of claims 2 to 4, **characterized in that** the material constituting the insulating portion (34, 134) is non-magnetic.

6. A magnetic plug according to any one of claims 1 to 5, **characterized in that** the first and second magnetic electrodes, and the intermediate electrode, have electrical terminals (30, 32, 40, 130, 132, 140) that are isolated from one another and that are designed to be connected to an electric circuit (22, 122).

7. A magnetic plug according to any one of claims 1 to 6, **characterized in that** the liquid circuit (14, 114) is an oil circuit.

8. Aircraft equipment provided with at least one magnetic plug according to any one of claims 1 to 7.

9. An aircraft engine (9) including an oil circuit fitted with a magnetic plug according to any one of claims 1 to 7.

## Patentansprüche

1. Magnetzapfen mit Signalfunktion (10, 110), dazu bestimmt, in einem Flüssigkeitskreislauf (14, 114) angeordnet zu werden, eine erste magnetische Elektrode (26, 126) und eine zweite magnetische Elektrode (28, 128) aufweisend, die voneinander isoliert sind und dazu bestimmt sind, in Kontakt mit der in dem Flüssigkeitskreislauf zirkulierenden Flüssigkeit zu sein, wenn der Magnetzapfen (10, 110) angebracht ist, um das Vorhandensein von Metallteilchen vom ferromagnetischen Typ (16, 116) zu detektieren, die möglicherweise von der Flüssigkeit transportiert werden, wobei der Zapfen **dadurch gekennzeichnet ist, daß** er außerdem eine vorzugsweise nichtmagnetische Zwischenelektrode (38, 138) aufweist, die zwischen der ersten und der zweiten magnetischen Elektrode angeordnet ist und ebenfalls dazu bestimmt ist, in Kontakt mit der in dem Flüssigkeitskreislauf zirkulierenden Flüssigkeit zu sein, wenn der Magnetzapfen angebracht ist, wodurch der Zapfen (10, 110) eine frühzeitige Detektion des Vorhandenseins solcher Metallteilchen ermöglicht.

2. Magnetzapfen nach Anspruch 1, **dadurch gekennzeichnet, daß** die erste und die zweite magnetische Elektrode (26, 126; 28, 128) durch einen isolierenden Abschnitt (34, 134) voneinander getrennt sind, und **dadurch**, daß die Zwischenelektrode (38, 138) mindestens teilweise in dem isolierenden Abschnitt (34, 134) angeordnet ist, derart, daß sie nicht mit der ersten und der zweiten magnetischen Elektrode in Kontakt steht.

3. Magnetzapfen nach Anspruch 2, **dadurch gekennzeichnet, daß** die Zwischenelektrode (38) auf dem Außenumfang des isolierenden Abschnitts (34) angeordnet ist.

4. Magnetzapfen nach Anspruch 2, **dadurch gekennzeichnet, daß** die erste und die zweite magnetische Elektrode (126, 128) sowie die Zwischenelektrode (138) die Form von Stäben aufweisen, die sich von dem isolierenden Abschnitt (134) aus erstrecken.

5. Magnetzapfen nach einem der Ansprüche 2 bis 4, **dadurch gekennzeichnet, daß** das Material, aus dem der isolierende Abschnitt (34, 134) besteht, nichtmagnetisch ist.

6. Magnetzapfen nach einem der Ansprüche 1 bis 5, **dadurch gekennzeichnet, daß** die erste und die zweite magnetische Elektrode sowie die Zwischenelektrode elektrische Anschlüsse (30, 32, 40, 130, 132, 140) umfassen, die voneinander isoliert sind und dazu bestimmt sind, an eine elektrische Schaltung (22, 122) angeschlossen zu werden.

7. Magnetzapfen nach einem der Ansprüche 1 bis 6, **dadurch gekennzeichnet, daß** der Flüssigkeitskreislauf (14, 114) ein Ölkreislauf ist.

8. Einrichtung für ein Luftfahrzeug, die mit mindestens einem Magnetzapfen nach einem der Ansprüche 1 bis 7 ausgestattet ist.

9. Motor (9) für ein Luftfahrzeug, der einen Ölkreislauf aufweist, der mit einem Magnetzapfen nach einem der Ansprüche 1 bis 7 ausgestattet ist.
